# EUROPÄISCHE PATENTSCHRIFT

(11) **EP 0 828 719 B1**
(45) Veröffentlichungstag und Bekanntmachung des Hinweises auf die Patenterteilung: **01.12.1999**
(21) Anmeldenummer: 96916134.8
(22) Anmeldetag: 20.05.1996
(51) Int. Cl.: C07D 249/12, A01N 43/653

(54) **MIKROBIZIDE (MERCAPTO-TRIAZOLYLMETHYL)-CYCLOPENTANOLE**
(MERCAPTO-TRIAZOLYLMETHYL) CYCLOPENTANOL MICROBICIDES
(MERCAPTO-TRIAZOLYLMETHYLE)-CYCLOPENTANOLS UTILISES COMME MICROBICIDES

(30) Priorität: 01.06.1995 DE 19520098
(43) Veröffentlichungstag der Anmeldung: 18.03.1998
(73) Patentinhaber: BAYER AG, 51368 Leverkusen (DE)
(72) Erfinder: JAUTELAT, Manfred, D-51399 Burscheid (DE); TIEMANN, Ralf, D-51375 Leverkusen (DE); DUTZMANN, Stefan, D-40721 Hilden (DE); STENZEL, Klaus, D-40595 Düsseldorf (DE)
(86) Internationale Anmeldenummer: EP9602162
(87) Internationale Veröffentlichungsnummer: WO9638423

(56) Entgegenhaltungen:
- EP-A- 0 251 086
- EP-A- 0 267 778

## Beschreibung

Die vorliegende Erfindung betrifft neue Triazolylmethyl-cyclopentanole, ein Verfahren zu deren Herstellung und deren Verwendung als Mikrobizide.

Es ist bereits bekannt geworden, daß zahlreiche Azolylmethyl-cyclopentan-Derivate fungizide Eigenschaften besitzen (vgl. EP-A 0 267 778). So läßt sich z.B. 5-(4-Chlor-benzyl)-2,2-dimethyl-1-(1,2,4-triazol-1-yl-methyl)-cyclopentan-1-ol zur Bekämpfung von Pilzen verwenden. Die Wirksamkeit dieses Stoffes ist gut, läßt aber bei niedrigen Aufwandmengen in manchen Fällen zu wünschen übrig.

Es wurden nun neue Triazolylmethyl-cyclopentanole der Formel 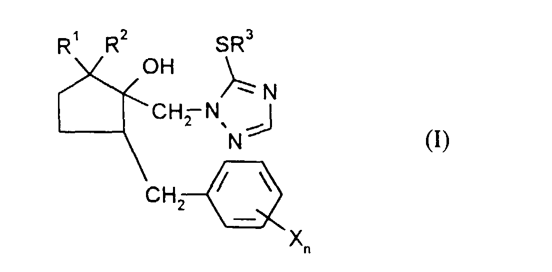 in welcher
- R¹ und R²: unabhängig voneinander für Wasserstoff oder Alkyl mit 1 bis 6 Kohlenstoffatomen stehen,
- R³: für Wasserstoff oder Alkyl mit 1 bis 4 Kohlenstoffatomen steht,
- X: für Halogen, Alkyl mit 1 bis 5 Kohlenstoffatomen, Alkoxy mit 1 bis 4 Kohlenstoffatomen, Phenyl, Phenoxy, Halogenalkyl mit 1 bis 4 Kohlenstoffatomen und 1 bis 5 Halogenatomen, Halogenalkoxy mit 1 bis 4 Kohlenstoffatomen und 1 bis 5 Halogenatomen oder für Halogenalkylthio mit 1 bis 4 Kohlenstoffatomen und 1 bis 5 Halogenatomen steht, und
- n: für die Zahlen 0; 1 oder 2 steht,
sowie deren Säureadditions-Salze und Metallsalz-Komplexe gefunden.

Die erfindungsgemäßen Stoffe enthalten mindestens zwei asymmetrisch substituierte Kohlenstoffatome und können deshalb in Form von Diastereomeren oder Enantiomeren anfallen. Die vorliegende Erfindung betrifft sowohl die einzelnen Isomeren als auch deren Gemische.

Weiterhin wurde gefunden, daß man Triazolylmethyl-cyclopentanole der Formel (I) sowie deren Säureadditions-Salze und Metallsalz-Komplexe erhält, wenn man Cyclopentanol-Derivate der Formel 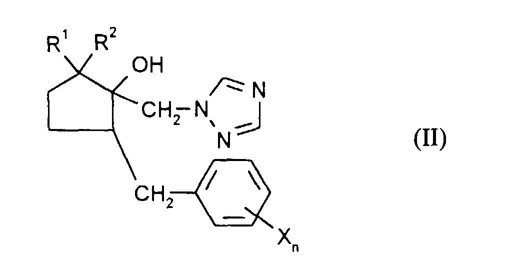 in welcher
- R¹, R², X und n: die oben angegebenen Bedeutungen haben,
nacheinander mit starken Basen und Schwefel in Gegenwart eines Verdünnungsmittels umsetzt und dann mit Wasser, gegebenenfalls in Gegenwart einer Säure hydrolysiert und gegebenenfalls die dabei entstehenden Verbindungen der Formel 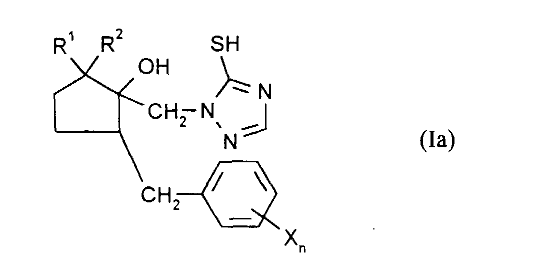 in welcher
- R¹, R², X und n: die oben angegebenen Bedeutungen haben,
mit Halogen-Verbindungen der Formel

R⁴-Hal (III)

in welcher
- R⁴: für Alkyl mit 1 bis 4 Kohlenstoffatomen steht und
- Hal: für Chlor, Brom oder Iod steht,
in Gegenwart eines Säurebindemittels und in Gegenwart eines Verdünnungsmittels umsetzt,
und gegebenenfalls anschließend an die so erhaltenen Verbindungen der Formel (I) eine Säure oder ein Metallsalz addiert.

Schließlich wurde gefunden, daß die neuen Triazolylmethyl-cyclopentanole der Formel (I) sowie deren Säureadditions-Salze und Metallsalz-Komplexe sehr gute mikrobizide Eigenschaften aufweisen und sowohl im Pflanzenschutz als auch im Materialschutz eingesetzt werden können.

Überraschenderweise besitzen die erfindungsgemäßen Stoffe eine bessere mikrobizide Wirksamkeit als die konstitutionell ähnlichsten, vorbekannten Verbindungen gleicher Wirkungsrichtung. So übertreffen die erfindungsgemäßen Stoffe das 5-(4-Chlor-benzyl)-2,2-dimethyl-1-(1,2,4-triazol-1-yl-methyl)-cyclopentan-1-ol bezüglich der fungiziden Eigenschaften.

Die erfindungsgemäßen Triazolylmethyl-cyclopentanole sind durch die Formel (I) allgemein definiert.
- R¹: steht vorzugsweise für Wasserstoff, Methyl, Ethyl, n-Propyl, Isopropyl, n-Butyl, iso-Butyl, sek.-Butyl, tert.-Butyl oder n-Pentyl.
- R²: steht vorzugsweise für Wasserstoff, Methyl, Ethyl, n-Propyl, Isopropyl, n-Butyl, iso-Butyl, sek.-Butyl, tert.-Butyl oder n-Pentyl.
- R³: steht vorzugsweise für Wasserstoff, Methyl oder Ethyl.
- X: steht vorzugsweise für Fluor, Chlor, Brom, Methyl, Ethyl, n-Propyl, Isopropyl, n-Butyl, iso-Butyl, sek.-Butyl, tert.-Butyl, Methoxy, Ethoxy, Phenyl, Phenoxy, Trichlormethyl, Trifluormethyl, Trifluormethoxy, Difluormethoxy oder Trifluormethylthio.
- n: steht auch vorzugsweise für die Zahlen 0, 1 oder 2. Wenn n für 2 steht, kann X für gleiche oder verschiedene Reste stehen.

Bevorzugte erfindungsgemäße Stoffe sind auch Additionsprodukte aus Säuren und denjenigen Triazolylmethyl-cyclopentanolen der Formel (I), in denen R¹, R², R³, X und n diejenigen Bedeutungen haben, die für diese Substituenten und diesen Index als bevorzugt genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsässure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure und 1,5-Naphthalindisulfonsäure, oder Camphersulfonsäure, Saccharin und Thiosaccharin.

Außerdem bevorzugte erfindungsgemäße Verbindungen sind Additionsprodukte aus Salzen von Metallen der II. bis IV- Haupt- und der 1. und II. sowie IV. bis VIII. Nebengruppe des Periodensystems der Elemente und denjenigen Triazolylmethyl-cyclopentanolen der Formel (1), in denen R', R², R³, X und n diejenigen Bedeutungen haben, die für diese Substituenten und diesen Index als bevorzugt genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Die erfindungsgemäßen Triazolylmethyl-cyclopentanole der Formel (I), in denen R³ für Wasserstoff steht, können in der "Mercapto"-Form der Formel 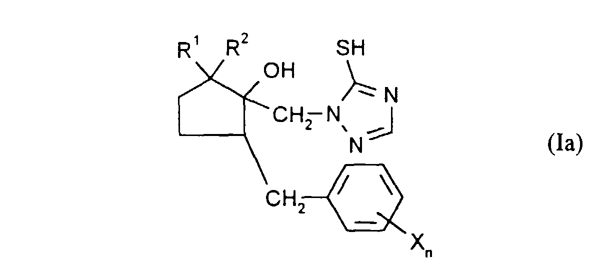 oder in der tautomeren "Thiono"-Form der Formel 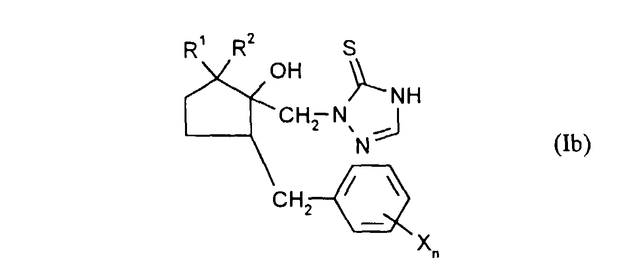 vorliegen. Der Einfachheit halber wird jeweils nur die "Mercapto"-Form aufgeführt.

Als Beispiele für erfindungsgemäße Stoffe seien die in der folgenden Tabelle aufgeführten Triazolylmethyl-cyclopentanole genannt.

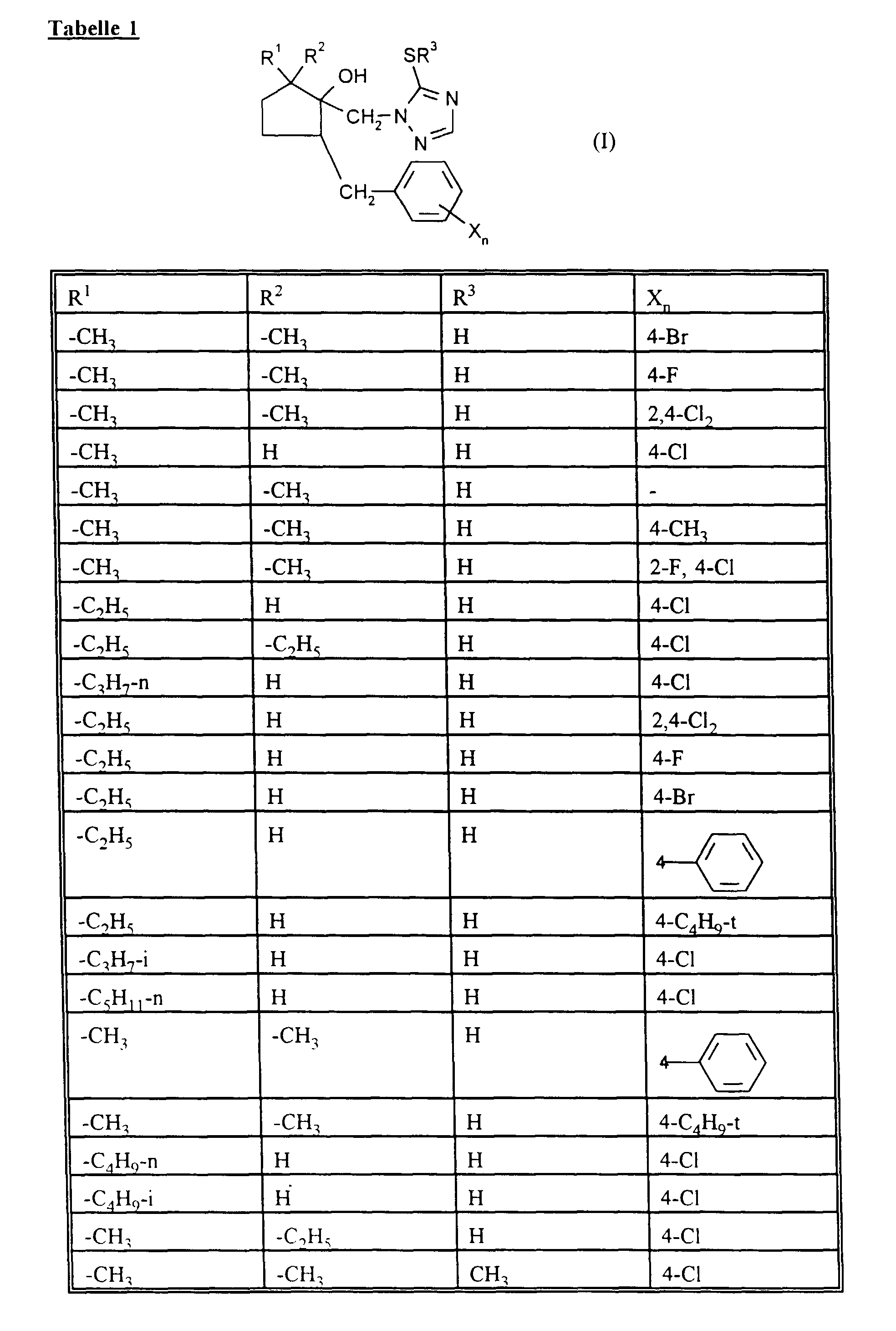

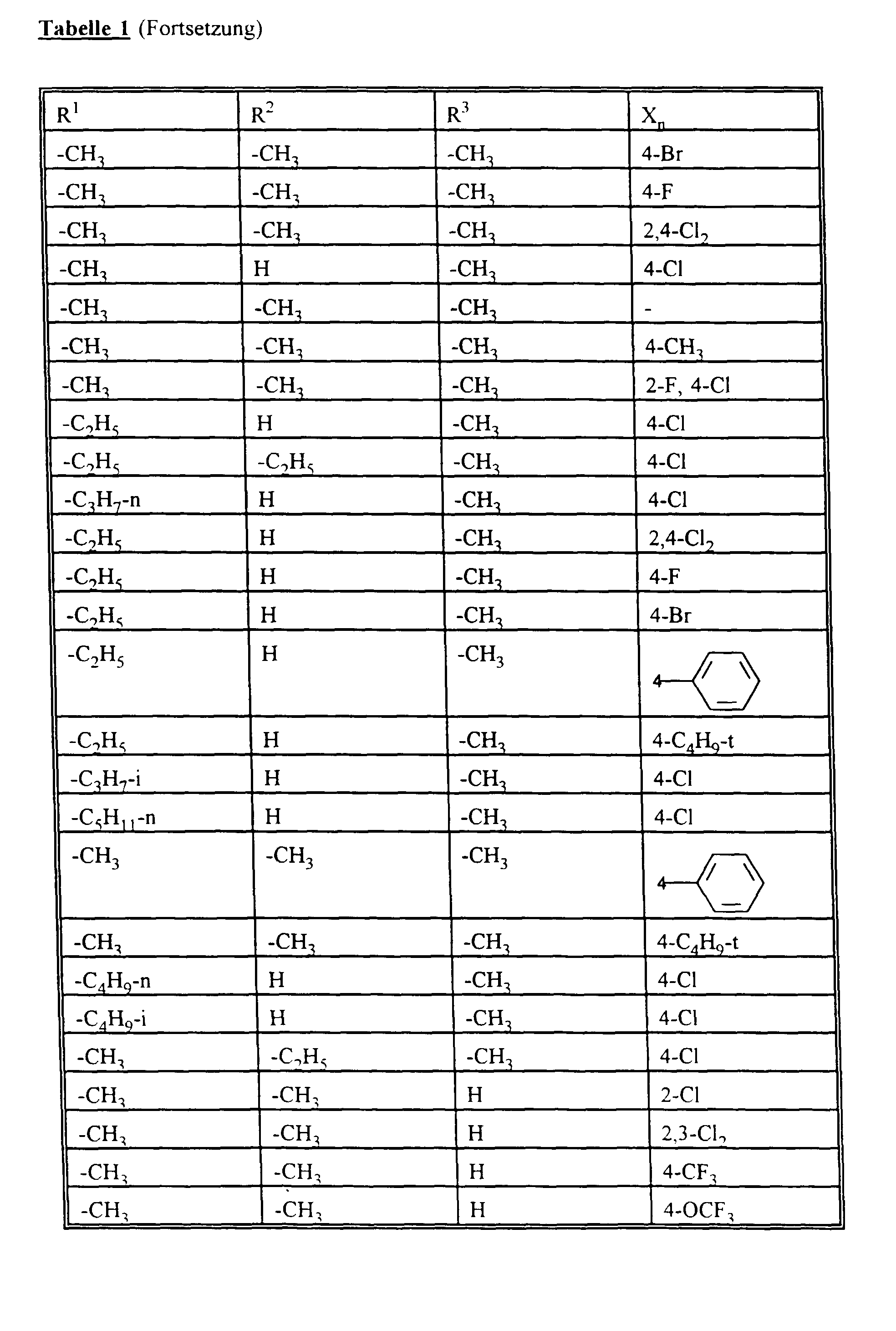

Verwendet man 5-(4-Chlor-benzyl)-2,2-dimethyl-1-(1,2,4-triazol-1-yl-methyl)cyclopentan-1-ol (Z-Form) als Ausgangsstoff, n-Butyl-lithium als starke Base und Schwefel-Pulver als Reaktionskomponente, so kann der Verlauf der ersten Stufe des erfindungsgemäßen Verfahrens durch das folgende Formelschema veranschaulicht werden:

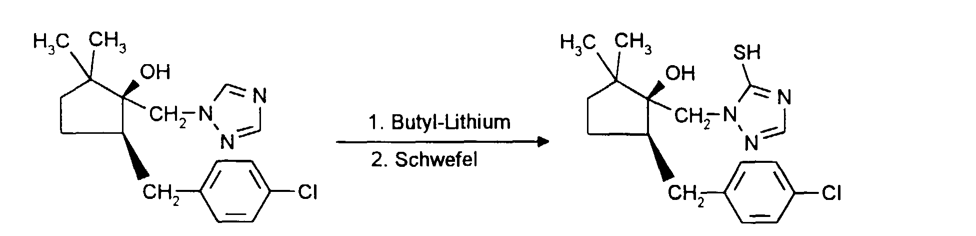

Verwendet man 5-(4-Chlor-benzyl)-2,2-dimethyl-1-(5-mercapto-1,2,4-triazol-1-ylmethyl)-cyclopentan-1-ol (Z-Form) als Ausgangsstoff und Methyliodid als Reaktionskomponente, so kann der Verlauf der zweiten Stufe des erfindungsgemäßen Verfahrens durch das folgende Formelschema veranschaulicht werden:

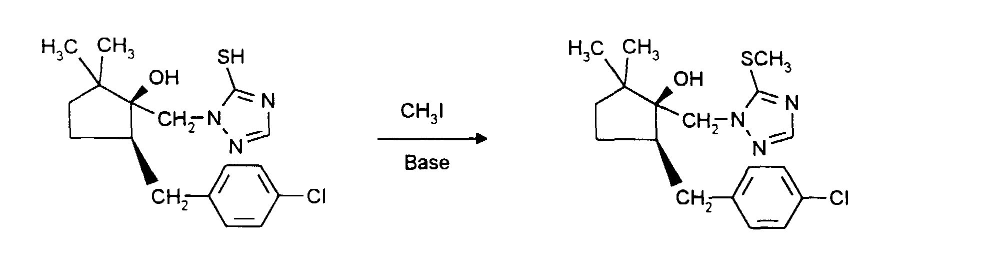

Die bei der Durchführung des erfindungsgemäßen Verfahrens als Ausgangsstoffe benötigten Cyclopentanol-Derivate sind durch die Formel (II) allgemein definiert. In dieser Formel haben R¹, R², X und n vorzugsweise diejenigen Bedeutungen, die bereits im Zusammenhang mit der Beschreibung der erfindungsgemäßen Stoffe der Formel (I) für diese Reste und diesen Index als bevorzugt genannt wurden.

Die Cyclopentanol-Derivate der Formel (II) sind bekannt oder lassen sich nach bekannten Methoden herstellen (vgl. EP-A 0 267 778).

Als Basen kommen bei der Durchführung der ersten Stufe des erfindungsgemäßen Verfahrens alle für derartige Reaktionen üblichen, starken Alkalimetall-Basen in Betracht. Vorzugsweise verwendbar sind n-Butyl-lithium, Lithium-diisopropylamid, Natriumhydrid, Natriumamid und auch Kalium-tert.-butylat im Gemisch mit Tetramethylethylen-diamin (= TMEDA).

Bei der Durchführung der ersten Stufe des erfindungsgemäßen Verfahrens kommen alle für derartige Umsetzungen üblichen inerten organischen Solventien als Verdünnungsmittel in Betracht. Vorzugsweise verwendbar sind Ether, wie Tetrahydrofuran, Dioxan, Diethylether und 1,2-Dimethoxyethan, ferner flüssiger Ammoniak oder auch stark polare Solventien, wie Dimethylsulfoxid.

Schwefel wird vorzugsweise in Form von Pulver eingesetzt. Zur Hydrolyse verwendet man bei der Durchführung der ersten Stufe des erfindungsgemäßen Verfahrens Wasser, gegebenenfalls in Gegenwart einer Säure. In Frage kommen hierbei alle für derartige Umsetzungen üblichen anorganischen oder organischen Säuren. Vorzugsweise verwendbar sind Essigsäure, verdünnte Schwefelsäure und verdünnte Salzsäure. Es ist jedoch auch möglich, die Hydrolyse mit wäßriger Ammoniumchlorid-Lösung durchzuführen.

Die Reaktionstemperaturen können bei der Durchführung der ersten Stufe des erfindungsgemäßen Verfahrens innerhalb eines bestimmten Bereiches variiert werden. Im allgemeinen arbeitet man bei Temperaturen zwischen -70°C und +20°C, vorzugsweise zwischen -70°C und 0°C.

Bei der Durchführung aller Schritte des erfindungsgemäßen Verfahrens arbeitet man im allgemeinen unter Normaldruck.

Bei der Durchführung der ersten Stufe des erfindungsgemäßen Verfahrens setzt man auf 1 Mol an Cyclopentanol-Derivat der Formel (II) im allgemeinen 2 bis 3 Äquivalente, vorzugsweise 2,0 bis 2,5 Äquivalente, an starker Base und anschliessend eine äquivalente Menge oder auch einen Überschuß an Schwefel ein. Die Umsetzung kann unter Schutzgas-atmosphäre, z.B. unter Stickstoff oder Argon, vorgenommen werden. Die Aufarbeitung erfolgt nach üblichen Methoden. Im allgemeinen geht man so vor, daß man das Reaktionsgemisch mit einem in Wasser wenig löslichen organischen Solvens extrahiert, die vereinigten organischen Phasen trocknet und einengt und den verbleibenden Rückstand gegebenenfalls durch Umkristallisation und/oder Chromatographie reinigt.

Die bei der Durchführung der zweiten Stufe des erfindungsgemäßen Verfahrens als Ausgangssubstanzen benötigten Verbindungen der Formel (Ia) sind erfindungsgemäße Stoffe.

Die bei der Durchführung des erfindungsgemäßen Verfahrens in der zweiten Stufe als Reaktionskomponenten benötigten Halogen-Verbindungen sind durch die Formel (III) allgemein definiert.
- R⁴: steht vorzugsweise für Methyl oder Ethyl.
- Hal: steht auch vorzugsweise für Chlor, Brom oder Iod.

Die Halogen-Verbindungen der Formel (III) sind bekannt.

Als Säurebindemittel kommen bei der Durchführung der zweiten Stufe des erfindungsgemäßen Verfahrens alle üblichen anorganischen oder organischen Basen in Frage. Vorzugsweise verwendbar sind Erdalkali- oder Alkalimetallhydroxide wie Natriumhydroxid, Calciumhydroxid, Kaliumhydroxid, oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Als Verdünnungsmittel kommen bei der Durchführung der zweiten Stufe des erfindungsgemäßen Verfahrens alle für derartige Umsetzungen üblichen, inerten organischen Solventien in Betracht. Vorzugsweise verwendbar sind Ether, wie Diethylether, Methyl-tert.-butyl-ether, Ethylenglykol-dimethylether, Tetrahydrofuran und Dioxan, ferner Nitrile, wie Acetonitril, und außerdem stark polare Solventien, wie Dimethylsulfoxid oder Dimethylformamid.

Die Reaktionstemperaturen können bei der Durchführung der zweiten Stufe des erfindungsgemäßen Verfahrens innerhalb eines größeren Bereiches variiert werden. Im allgemeinen arbeitet man bei Temperaturen zwischen 0°C und 120°C, vorzugsweise zwischen 20°C und 100°C.

Bei der Durchführung der zweiten Stufe des erfindungsgemäßen Verfahrens setzt man auf 1 Mol an Triazolylmethyl-cyclopentanol der Formel (Ia) im allgemeinen 1 bis 2 Mol an Halogen-Verbindung der Formel (III) sowie eine äquivalente Menge oder auch einen Überschuß an Säurebindemittel ein. Die Aufarbeitung erfolgt nach üblichen Methoden. Im allgemeinen geht man so vor, daß man das Reaktionsgemisch mit wäßriger Base und einem mit Wasser wenig mischbaren organischen Lösungsmittel versetzt, die organische Phase abtrennt, trocknet und einengt. Das erhaltene Produkt kann gegebenenfalls nach üblichen Methoden, z.B. durch Umkristallisation, von noch vorhandenen Verunreinigungen befreit werden.

Die nach dem erfindungsgemäßen Verfahren erhältlichen Triazolylmethyl-cyclopentanole der Formel (I) können in Säureadditions-Salze oder Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (1). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung unerwünschter Mikroorganismen, wie Fungi und Bakterien, im Pflanzenschutz und im Materialschutz eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubensis;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Pyricularia oryzae und Pellicularia sasakii an Reis sowie zur Bekämpfung von Getreidekrankheiten, wie Pseudocercosporella, Erysiphe- und Fusarium-Arten. Außerdem lassen sich die erfindungsgemäßen Stoffe sehr gut gegen Venturia und Sphaerotheca einsetzen. Sie besitzen darüber hinaus auch eine sehr gute in-vitro Wirkung.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in übliche Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel wie Alkohole als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe, sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/ oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolygylkol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Pflanzenschutz im allgemeinen zwischen 0,1 und 95 Gewichtsprozent Wirkstoff, vorzugsweise zwischen 0,5 und 90 %.

Die erfindungsgemäßen Wirkstoffe können bei Verwendung im Pflanzenschutz in den Formulierungen in Mischung mit bekannten Fungiziden, Bakteriziden, Akariziden, Nematiziden oder Insektiziden eingesetzt werden, um so z.B. das Wirkungsspektrum zu verbreitern oder Resistenzentwicklungen vorzubeugen. In manchen Fällen treten auch synergistische Effekte auf.

Für die Mischungen kommen beispielsweise folgende Stoffe in Frage.
Fungizide:
   2-Aminobutan; 2-Anilino-4-methyl-6-cyclopropyl-pyrimidin; 2',6'-Dibromo-2-methyl-4'-trifluoromethoxy-4'-trifluoro-methyl-1 ,3-thiazol-5-carboxanilid; 2,6-Dichloro-N-(4-trifluoromethylbenzyl)-benzamid; (E)-2-Methoximino-N-methyl-2-(2-phenoxyphenyl)-acetamid; 8-Hydroxychinolinsulfat; Methyl-(E)-2--{2-[6-(2-cyanophenoxy)-pyrimidin-4-yloxy]-phenyl}-3-methoxyacrylat; Methyl-(E)-methoximino [alpha-(o-tolyloxy)-o-tolyl]-acetat; 2-Phenylphenol (OPP), Aldimorph, Ampropylfos, Anilazin, Azaconazol,
   Benalaxyl, Benodanil, Benomyl, Binapacryl, Biphenyl, Bitertanol, Blasticidin-S, Bromuconazole, Bupirimate, Buthiobate,
   Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
   Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
   Edifenphos, Epoxyconazole, Ethirimol, Etridiazol,
   Fenarimol, Fenbuconazole, Fenfuram, Fenitropan, Fenpiclonil, Fenpropidin, Fenpropimorph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
   Guazatine,
   Hexachlorobenzol, Hexaconazol, Hymexazol,
   Imazalil, lmibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
   Mancopper, Mancozeb, Maneb, Mepanipyrim, Mepronil, Metalaxyl, Metconazol, Methasulfocarb, Methfuroxam, Metiram, Metsulfovax, Myclobutanil,
   Nickel-dimethyldithiocarbamat, Nitrothal-isopropyl, Nuarimol,
   Ofurace, Oxadixyl, Oxamocarb, Oxycarboxin,
   Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
   Quintozen (PCNB),
   Schwefel und Schwefel-Zubereitungen,
   Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
   Validamycin A, Vinclozolin,
   Zineb, Ziram.
**Bakterizide:**
   Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
   Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
   Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
   Dimethylvinphos, Dioxathion, Disulfoton,
   Edifenphos, Emamectin, Esfenvalerat, Ethiofencarb, Ethion, Ethofenprox, Ethoprophos, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivermectin, Lambda-cyhalothrin, Lufenuron,
   Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, NI 25, Nitenpyram
   Omethoat, Oxamyl, Oxydemethon M, Oxydeprofos,
   Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen, Quinalphos,
   RH 5992,
   Salithion, Sebufos, Silafluofen, Sulfotep, Sulprofos,
   Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
   Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die zum Schutz technischer Materialien verwendeten Mittel enthalten die Wirkstoffe im allgemeinen in einer Menge von 1 bis 95%, bevorzugt von 10 bis 75 %.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen errmittelt werden. Im allgemeinen liegen die Anwendungskonzentrationen im Bereich von 0,001 bis 5 Gewichts-%, vorzugsweise von 0,05 bis 1,0 Gewichts-% bezogen auf das zu schützende Material.

Die Wirksamkeit und das Wirkungsspektrum der erfindungsgemäß im Materialschutz zu verwendenden Wirkstoffe bzw. der daraus herstellbaren Mittel, Konzentrate oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäßen Verbindungen.

Die Herstellung und die Verwendung der erfindungsgemäßen Stoffe gehen aus den folgenden Beispielen hervor.

### Herstellungsbeispiele

### Beispiel 1

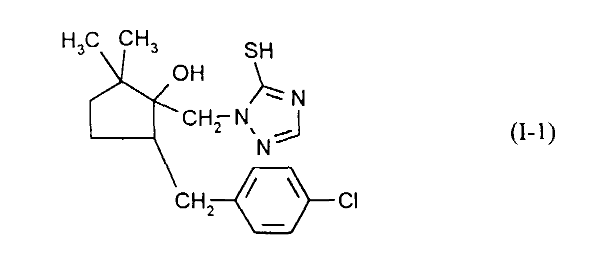

Ein Gemisch aus 1,6 g (5 mmol) 5-(4-Chlorbenzyl)-2,2-dimethyl-1-(1,2,4-triazol-1-yl-methyl)-cyclopentan-1-ol (Z-Form) und 30 ml absolutem Tetrahydrofuran wird bei -20°C mit 4 ml (10 mmol) n-Butyl-lithium in Hexan versetzt und 30 Minuten bei 0°C nachgerührt. Anschließend wird das Reaktionsgemisch auf -70°C abgekühlt, unter Rühren mit 0,19 g (6 mmol) Schwefel-Pulver versetzt, dann 1 Stunde bei -70°C und danach 2 Stunden bei 0°C gerührt. Man verdünnt das entstehende Gemisch mit Essigsäureethylester und schüttelt mehrfach mit gesättigter, wäßriger Ammoniumchlorid-Lösung aus. Die organische Phase wird über Natriumsulfat getrocknet und dann unter vermindertem Druck eingeengt. Das anfallende Rohprodukt (2,0 g) wird aus Toluol umkristallisiert. Man erhält auf diese Weise 1,1 g (63 % der Theorie) an 5-(4-Chlor-benzyl)-2,2-dimethyl-1-(5-mercapto-1,2,4-triazol-1-yl-methyl)-cyclopentan-1-ol (Z-Form) als Festsubstanz vom Schmelzpunkt 179 bis 180°C.
GC/MS (ci): 352 (M+H⁺)

### Verwendungsbeispiele

In den folgenden Verwendungsbeispielen wurde die nachstehend aufgeführte Verbindung als Vergleichssubstanz eingesetzt: 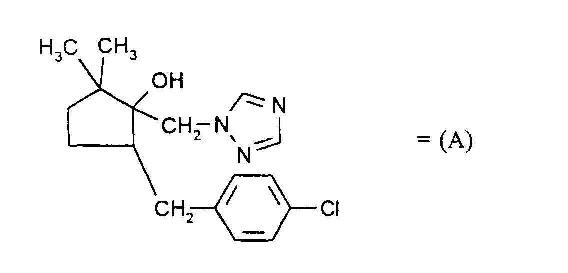 (Z-Form)
Bekannt aus EP-A 0 267 778

### Beispiel A

| Venturia-Test (Apfel) / protektiv | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20C und einer relativen Luftfeuchtigkeit von ca. 70 % aufgestellt.

12 Tage nach der Inokulation erfolgt die Auswertung. Dabei bedeutet 0 % einen Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

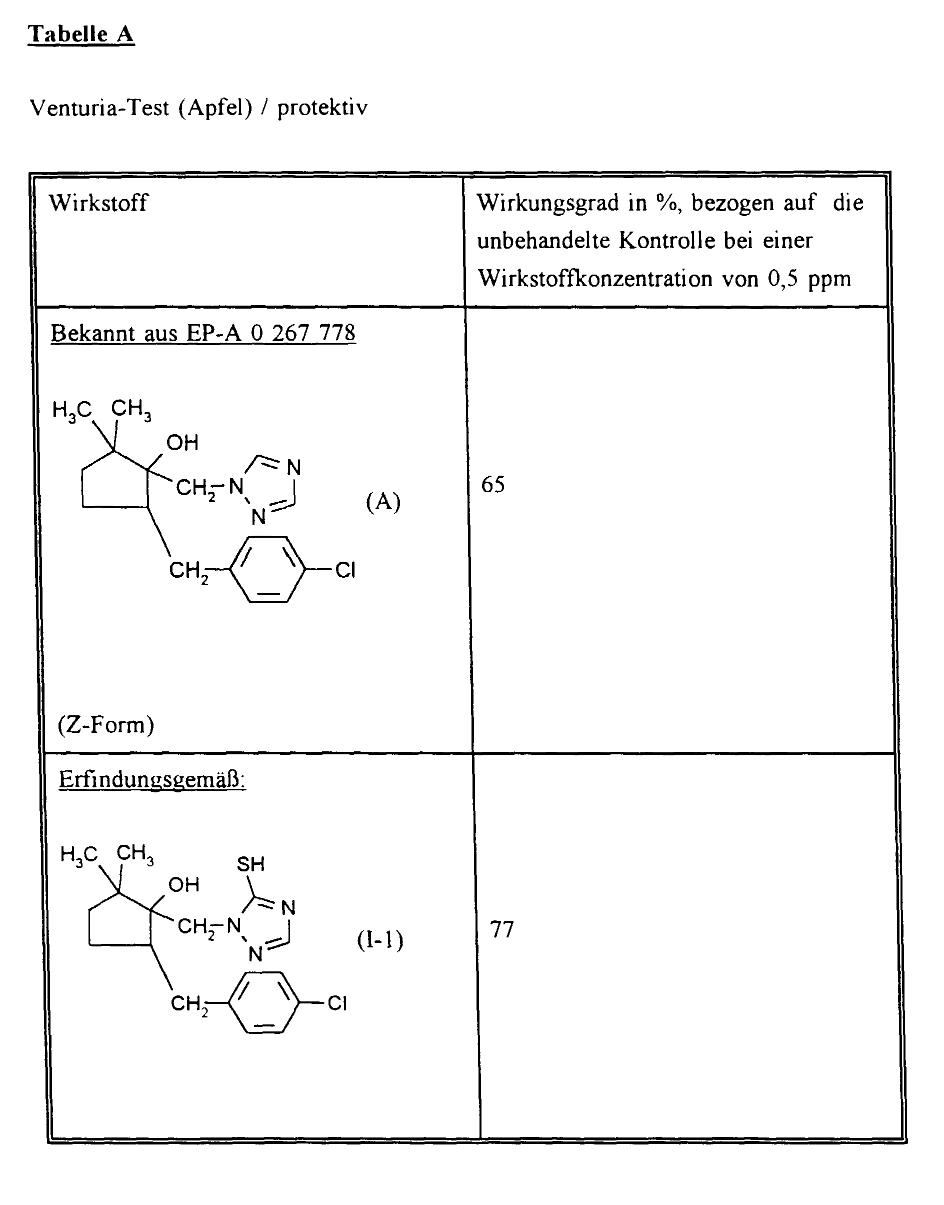

### Beispiel B

| Podosphaera-Test (Apfel) / protektiv | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen durch Bestäuben mit Konidien des Apfelmehltauerregers (Podosphaera leucotricha) inokuliert.

Die Pflanzen werden dann im Gewächshaus bei 23°C und einer relativen Luftfeuchtigkeit von ca. 70 % aufgestellt.

10 Tage nach der Inokulation erfolgt die Auswertung. Dabei bedeutet 0 % einen Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

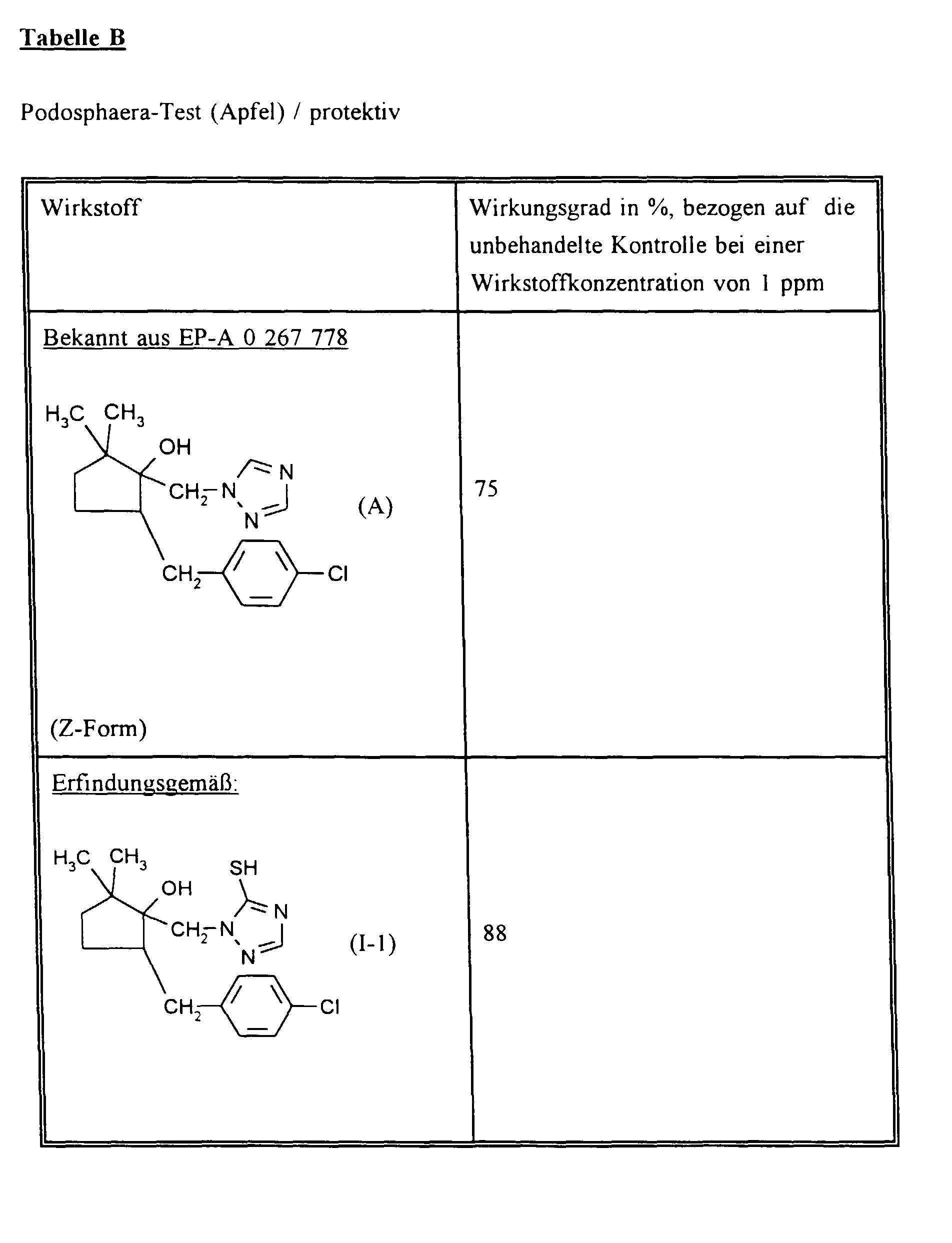

## Patentansprüche

1. Triazolylmethyl-cyclopentanole der Formel 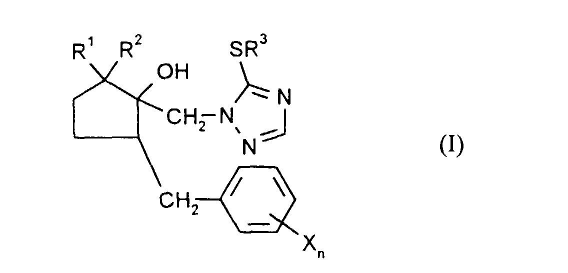 in welcher
R¹ und R³ unabhängig voneinander für Wasserstoff oder Alkyl mit 1 bis 6 Kohlenstoffatomen stehen,
R³ für Wasserstoff oder Alkyl mit 1 bis 4 Kohlenstoffatomen steht,
X für Halogen, Alkyl mit 1 bis 5 Kohlenstoffatomen, Alkoxy mit 1 bis 4 Kohlenstoffatomen, Phenyl, Phenoxy, Halogenalkyl mit 1 bis 4 Kohlenstoffatomen und 1 bis 5 Halogenatomen, Halogenalkoxy mit 1 bis 4 Kohlenstoffatomen und 1 bis 5 Halogenatomen oder für Halogenalkylthio mit 1 bis 4 Kohlenstoffatomen und 1 bis 5 Halogenatomen steht, und
n für die Zahlen 0, 1 oder 2 steht,
sowie deren Säureadditions-Salze und Metallsalz-Komplexe.

2. Triazolylmethyl-cyclopentanole der Formel (I) gemäß Anspruch 1, in denen
R¹ für Wasserstoff, Methyl, Ethyl, n-Propyl, Isopropyl, n-Butyl, iso-Butyl, sek.-Butyl, tert.-Butyl oder n-Pentyl steht,
R² für Wasserstoff, Methyl, Ethyl, n-Propyl, Isopropyl, n-Butyl, iso-Butyl, sek.-Butyl, tert.-Butyl oder n-Pentyl steht,
R³ für Wasserstoff, Methyl oder Ethyl steht,
X für Fluor, Chlor, Brom, Methyl, Ethyl, n-Propyl, Isopropyl, n-Butyl, iso-Butyl, sek.-Butyl, tert.-Butyl, Methoxy, Ethoxy, Phenyl, Phenoxy, Trichlormethyl, Trifluormethyl, Trifluormethoxy, Difluormethoxy oder Trifluormethylthio steht, und
n für die Zahlen 0, 1 oder 2 steht, wobei X für gleiche oder verschiedene Reste steht, wenn n für 2 steht.

3. Verfahren zur Herstellung von Triazolylmethyl-cyclopentanolen der Formel (I) gemäß Anspruch 1 sowie von deren Säureadditionssalzen und Metallsalz-Komplexen, dadurch gekennzeichnet, daß man Cyclopentanol-Derivate der Formel 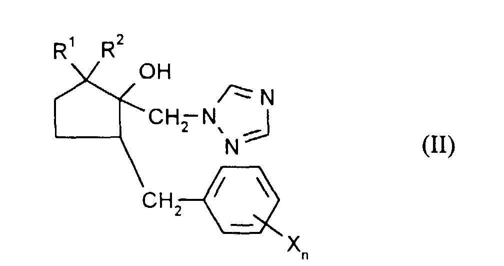 in welcher
R¹, R² X und n die oben angegebenen Bedeutungen haben,
nacheinander mit starken Basen und Schwefel in Gegenwart eines Verdünnungsmittels umsetzt und dann mit Wasser, gegebenenfalls in Gegenwart einer Säure hydrolysiert und gegebenenfalls die dabei entstehenden Verbindungen der Formel 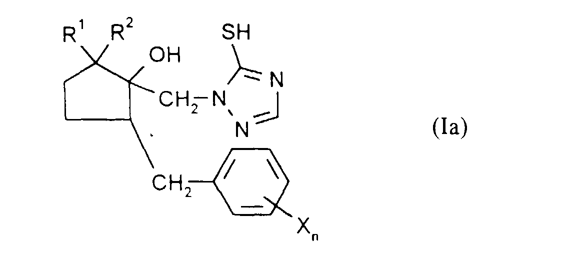 in welcher
R¹, R², X und n die oben angegebenen Bedeutungen haben,
mit Halogen-Verbindungen der Formel
R⁴-Hal (III)
in welcher
R⁴ für Alkyl mit 1 bis 4 Kohlenstoffatomen steht und
Hal für Chlor, Brom oder Iod steht,
in Gegenwart eines Säurebindemittels und in Gegenwart eines Verdünnungsmittels umsetzt,
und gegebenenfalls anschließend an die so erhaltenen Verbindungen der Formel (I) eine Säure oder ein Metallsalz addiert.

4. Mikrobizide Mittel, gekennzeichnet durch einen Gehalt an mindestens einem Triazolylmethyl-cyclopentanol der Formel (I) gemäß Anspruch 1 bzw. an einem Säureadditionssalz oder Metallsalz-Komplex eines Triazolylmethyl-cyclopentanoles der Formel (1).

5. Verwendung von Triazolylmethyl-cyclopentanolen der Formel (I) gemäß Anspruch 1 bzw. von deren Säureadditionssalzen oder Metallsalz-Komplexen als Mikrobizide im Pflanzenschutz und im Materialschutz.

6. Verfahren zur Bekämpfung von unerwünschten Mikroorganismen im Pflanzenschutz und im Materialschutz, dadurch gekennzeichnet, daß man Triazolylmethyl-cyclopentanole der Formel (I) gemäß Anspruch 1 bzw. deren Säureadditionssalze oder Metallsalz-Komplexe auf die Mikroorganismen und/oder deren Lebensraum ausbringt.

7. Verfahren zur Herstellung von mikrobiziden Mitteln, dadurch gekennzeichnet, daß man Triazolylmethyl-cyclopentanole der Formel (I) gemäß Anspruch 1 bzw. deren Säureadditionssalze oder Metallsalz-Komplexe mit Streckmitteln und/oder oberflächenaktiven Stoffen vermischt.

## Claims

1. Triazolylmethyl-cyclopentanols of the formula 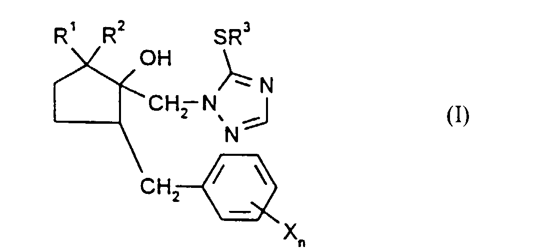 in which
R¹ and R² independently of one another represent hydrogen or alkyl having 1 to 6 carbon atoms,
R³ represents hydrogen or alkyl having 1 to 4 carbon atoms,
X represents halogen, alkyl having 1 to 5 carbon atoms, alkoxy having 1 to 4 carbon atoms, phenyl, phenoxy, halogenoalkyl having 1 to 4 carbon atoms and 1 to 5 halogen atoms, halogenoalkoxy having 1 to 4 carbon atoms and 1 to 5 halogen atoms, or halogenoalkylthio having 1 to 4 carbon atoms and 1 to 5 halogen atoms, and
n represents the numbers 0, 1 or 2,
and their acid addition salts and metal salt complexes.

2. Triazolylmethyl-cyclopentanols of the formula (I) according to Claim 1 in which
R¹ represents hydrogen, methyl, ethyl, n-propyl, isopropyl, n-butyl, isobutyl, sec-butyl, tert-butyl or n-pentyl
R² represents hydrogen, methyl, ethyl, n-propyl, isopropyl, n-butyl, isobutyl, sec-butyl, tert-butyl or n-pentyl
R³ represents hydrogen, methyl or ethyl,
X represents fluorine, chlorine, bromine, methyl, ethyl, n-propyl, isopropyl, n-butyl, isobutyl, sec-butyl, tert-butyl, methoxy, ethoxy, phenyl, phenoxy, trichloromethyl, trifluoromethyl, trifluoromethoxy, difluoromethoxy or trifluoromethylthio, and
n represents the numbers 0, 1 or 2, X representing identical or different radicals if n represents 2.

3. Process for the preparation of triazolylmethyl-cyclopentanols of the formula (I) according to Claim 1 and of their acid addition salts and metal salt complexes, characterized in that cyclopentanol derivatives of the formula 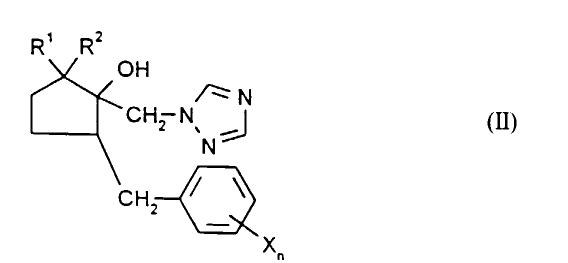 in which
R¹, R², X and n have the abovementioned meanings
are reacted in succession with strong bases and sulphur in the presence of a diluent, the product is then hydrolysed with water, if appropriate in the presence of an acid, and, if appropriate, the resulting compounds of the formula 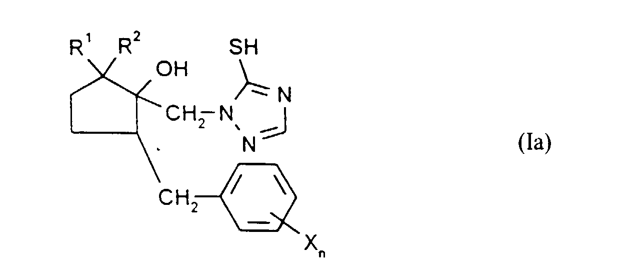 in which
R¹, R², X and n have the abovementioned meanings,
are reacted with halogen compounds of the formula
R⁴-Hal (III)
in which
R⁴ represents alkyl having 1 to 4 carbon atoms,
Hal represents chlorine, bromine or iodine
in the presence of an acid-binding agent and in the presence of a diluent,
and, if appropriate, an acid or a metal salt is subjected to an addition reaction with the resulting compounds of the formula (I).

4. Microbicidal compositions, characterized in that they comprise at least one triazolylmethyl-cyclopentanol of the formula (I) according to Claim 1 or an acid addition salt or metal salt complex of a triazolylmethyl-cyclopentanol of the formula (I).

5. Use of triazolylmethyl-cyclopentanols of the formula (I) according to Claim 1 and of their acid addition salts or metal salt complexes as microbicides in plant protection and in the protection of materials.

6. Method of combating undesired microorganisms in plant protection and in the protection of materials, characterized in that triazolylmethyl-cyclopentanols of the formula (I) according to Claim 1 or their acid addition salts or metal salt complexes are applied to the microorganisms and/or their environment.

7. Process for the preparation of microbicidal compositions, characterized in that triazolylmethyl-cyclopentanols of the formula (I) according to Claim 1 or their acid addition salts or metal salt complexes are mixed with extenders and/or surface-active substances.

## Revendications

1. Triazolylméthyl-cyclopentanols de formule 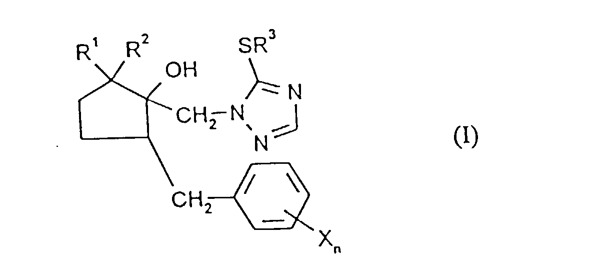 dans laquelle
R¹ et R² représentent, indépendamment l'un de l'autre, l'hydrogène ou un groupe alkyle ayant 1 à 6 atomes de carbone,
R³ est l'hydrogène ou un groupe alkyle ayant 1 à 4 atomes de carbone,
X est un halogène, un groupe alkyle ayant 1 à 5 atomes de carbone, alkoxy ayant 1 à 4 atomes de carbone, phényle, phénoxy, halogénalkyle ayant 1 à 4 atomes de carbone et 1 à 5 atomes d'halogène, halogénalkoxy ayant 1 à 4 atomes de carbone et 1 à 5 atomes d'halogène ou un groupe halogénalkylthio ayant 1 à 4 atomes de carbone et 1 à 5 atomes d'halogène, et
n représente les nombres 0, 1 ou 2,
ainsi que leurs sels d'addition d'acides et leurs complexes avec des sels métalliques.

2. Triazolyméthyl-cyclopentanols de formule (I) suivant la revendication 1, dans lesquels
R¹ est l'hydrogène, un groupe méthyle, éthyle, n-propyle, isopropyle, n-butyle, isobutyle, sec.-butyle, tertio-butyle ou n-pentyle,
R² est l'hydrogène, un groupe méthyle, éthyle, n-propyle, isopropyle, n-butyle, isobutyle, sec.-butyle, tertio-butyle ou n-pentyle,
R³ est l'hydrogène, un groupe méthyle ou éthyle,
X est le fluor, le chlore, le brome, un groupe méthyle, éthyle, n-propyle, isopropyle, n-butyle, isobutyle, sec.-butyle, tertio-butyle, méthoxy, éthoxy, phényle, phénoxy, trichlorométhyle, trifluorométhyle, trifluorométhoxy, difluorométhoxy ou trifluorométhylthio et
n représente les nombres 0, 1 ou 2, X désignant des restes identiques ou différents lorsque n est égal à 2.

3. Procédé pour la production de triazolylméthyl-cyclopentanols de formule (I) suivant la revendication 1 ainsi que de leurs sels d'addition d'acides et de leurs complexes avec des sels métalliques, caractérisé en ce qu'on fait réagir des dérivés de cylcopentanols de formule 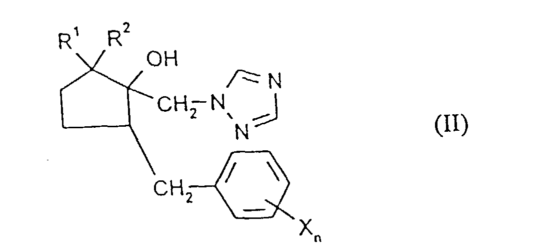 dans laquelle
R¹, R², X et n ont les définitions indiquées ci-dessus,
successivement avec des bases fortes et du soufre en présence d'un diluant puis on effectue l'hydrolyse avec de l'eau, le cas échéant en présence d'un acide et on fait réagir éventuellement les composés ainsi formés, de formule 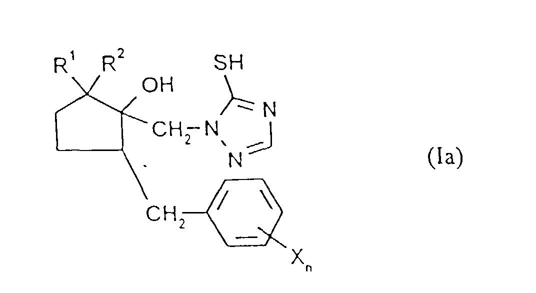 dans laquelle
R¹, R², X et n ont les définitions indiquées ci-dessus,
avec des composés halogénés de formule
R⁴-Hal (III)
dans laquelle
R⁴ est un groupe alkyle ayant 1 à 4 atomes de carbone et
Hal représente le chlore, le brome ou l'iode,
en présence d'un accepteur d'acide et en présence d'un diluant,
après quoi, le cas échéant, on additionne sur les composés de formule (I) ainsi obtenus un acide ou un sel métallique.

4. Compositions microbicides, caractérisées par une teneur en au moins un triazolylméthycyclopentanol de formule (I) suivant la revendication 1 ou d'un sel d'addition d'acide ou d'un complexe avec un sel métallique d'un triazolylméthyl-cyclopentanol de formule (I).

5. Utilisation de triazolylméthyl-cyclopentanols de formule (I) suivant la revendication 1 ou de leurs sels d'addition d'acides ou de leurs complexes avec des sels métalliques comme microbicides dans la protection de plantes et dans la protection des matériaux.

6. Procédé pour combattre des micro-organismes indésirables dans la protection des plantes et dans la protection des matériaux, caractérisé en ce qu'on fait agir les triazolylméthyl-cyclopentanols de formule (I) suivant la revendication 1 ou leurs sels d'addition d'acides ou leurs complexes avec des sels métalliques sur les micro-organismes et/ou sur leurs milieux.

7. Procédé de préparation de compositions microbicides, caractérisé en ce qu'on mélange avec des diluants et/ou des agents tensio-actifs des triazolylméthyl-cyclopentanols de formule (I) suivant la revendication 1 ou leurs sels d'addition d'acides ou leurs complexes avec des sels métalliques.
